# EUROPEAN PATENT SPECIFICATION

(11) **EP 2 734 241 B1**
(45) Date of publication and mention of the grant of the patent: **01.03.2017**
(21) Application number: 12745521.0
(22) Date of filing: 11.07.2012
(51) Int. Cl.: A61L 2/18, A61L 11/00

(54) **EQUIPMENT FOR DISINFECTING ORGANIC SUBSTANCES AND METHOD SUITED TO CARRY OUT SAID DISINFECTION**
VORRICHTUNG ZUR DESINFEKTION VON ORGANISCHEN STOFFEN UND GEEIGNETES VERFAHREN ZUR DURCHFÜHRUNG DIESER DESINFEKTION
EQUIPEMENT PERMETTANT DE DÉSINFECTER DES SUBSTANCES ORGANIQUES ET PROCÉDÉ APTE À RÉALISER LADITE DÉSINFECTION

(30) Priority: 22.07.2011 IT VI20110199
(43) Date of publication of application: 28.05.2014
(73) Proprietor: IN.CAS. S.r.l. (Innovazioni Casamichele), 37062 Dossobuono di Villafranca (IT)
(72) Inventor: CASAMICHELE, Gianni, I-37012 Bussolengo (VR) (IT)
(74) Representative: Ziliotto, Tiziano
(86) International application number: PCT/IB2012/001387
(87) International publication number: WO 2013/014506

(56) References cited:
- EP-A1- 1 757 313
- WO-A1-00/34192
- WO-A1-96/26750
- WO-A1-99/00154
- WO-A1-03/026724
- WO-A1-2007/125100
- GB-A- 2 251 433

## Description

### TECHNICAL FIELD OF THE INVENTION

The present invention concerns the treatment of organic substances, and in particular disinfection operations for potentially infected organic substances. More particularly, the present invention concerns the disinfection treatment of potentially infected organic substances constituted, for example, by the liquid residues of surgical operations.

The present invention also concerns a method for treating said organic substances.

### DESCRIPTION OF THE STATE OF THE ART

It is known that in hospitals it is necessary to maintain a hygienic and healthy environment for the purpose of preventing the spreading of diseases or infections, for example in wards and operating theatres.

A much felt need concerns the correct treatment of all those substances that can be infected, for example all the organic liquids resulting from surgical operations. During surgical operations said liquids, typically blood and/or urine, are duly collected by means of a suitable collection bag and then conveyed into special disposable storage containers.

Said liquids can potentially be infected, for example by bacteria carrying more or less dangerous and infectious diseases, like for example hepatitis B, hepatitis C, HIV, HBV, HCV. The organic liquids must therefore be handled with extreme care by the operators, in order to avoid any type of contamination and/or contact, both direct and with the surrounding environment.

The liquids introduced in the disposable storage containers are then transported to special disposal centres to be treated as special waste.

All the potentially dangerous liquid substances are treated in the same manner, for example the urine collected in hospital wards, especially where bladder cleaning is carried out.

Said collection and storage operations require, therefore, a high degree of attention by the operators, in order to ensure the highest possible level of environmental and personal hygiene.

However, the operations described above pose some drawbacks.

A first drawback derives from the risk of contact with potentially infected organic liquids before they are put in the disposable storage container.

Another drawback lies in that so long as the disposable storage containers are not collected by the operators in charge with disposal, they remain inconveniently located in hospital environments, with their potentially infected contents.

A further drawback is represented by the space occupied, which is due to the need for continuous availability of empty containers ready to be filled with organic liquids.

Another yet not the least drawback is represented by the high disposal costs to be born for said organic liquids, since they are considered special waste.

Documents GB 2 251 433 A and WO 99/00154 disclose equipments and methods for disinfecting liquid medical wastes.

The main object of the present invention is thus to solve or at least partially overcome the problems that characterize the solutions known in the state of the art.

In particular, it is the object of the present invention to ensure that the organic substance collection and disinfection operations are as safe as possible.

It is another object of the present invention to reduce the risk of the personnel coming into contact with potentially infected organic substances.

It is a further object of the present invention to propose a solution that makes it possible to simplify the operations needed for the disposal of the organic substances produced in hospital environments.

It is another object of the present invention to reduce to a minimum the time that elapses from the moment when the organic substance is collected to the moment when it is disinfected.

It is a further object of the present invention to reduce the costs deriving from the treatment of organic substances compared to the solutions known in the art.

### SUMMARY OF THE PRESENT INVENTION

The present invention is based on the general consideration that the problems found in the state of the art can be at least partly overcome by providing a piece of equipment for the disinfection of organic substances that carries out said disinfection on site.

The words "organic substance" in the text below will preferably mean a liquid substance. Said liquid substance can be thicker or thinner, as well as containing larger or smaller suspended particles.

The subject of the present invention is thus a piece of equipment for disinfecting an organic substance, as defined in claim 1.

The equipment comprises means for the intake of the organic substance associated with the conveying inlet.

Advantageously, the equipment comprises means for detecting the contents inside the main containment means.

The means for conveying the disinfecting substance preferably comprise a peristaltic pump.

The equipment properly comprises discharging means associated with the main containment means for discharging the contents of the main containment means. In a preferred embodiment of the invention, the equipment comprises conveying means of liquid substances, associated with the main containment means.

The equipment advantageously comprises recirculation means associated with the main containment means for the recirculation of the substances contained in the main containment means.

In a preferred embodiment of the invention, the recirculation means cooperate with the means for the intake of the organic substance.

The recirculation means properly comprise a diffuser element arranged inside the main containment means.

In a preferred embodiment of the invention, the equipment comprises auxiliary containment means suited to contain a cleaning and disinfecting substance for disinfecting the equipment.

The equipment comprises a logic control unit.

In practice, the logic control unit is properly connected to the means for conveying the disinfecting substance.

In a preferred embodiment of the invention the equipment comprises mixing means associated with the main containment means.

The equipment preferably comprises a containment body and wheeled means associated with the body.

Advantageously, the equipment comprises a display for displaying and/or inputting data.

A second aspect of the invention concerns a method for disinfecting an organic substance, as defined in claim 9.

Preferably, the step of maintaining the organic substance and the dose of disinfecting substance in contact takes place at least for a predetermined minimum time of action.

Advantageously, the step of adding a dose of a disinfecting substance comprises the step of checking the percentage ratio between the quantity of the added dose of the disinfecting substance with respect to the organic substance to be disinfected, in such a way as to maintain said ratio above a predetermined minimum value.

In a preferred embodiment of the invention, the method comprises a step of introducing a given quantity of a filling substance in the container in order to obtain a mixture.

Advantageously, the method comprises a further step of adding a dose of a disinfecting substance in the container and a step of checking the percentage ratio between the quantity of the added dose of disinfecting substance in relation to the mixture.

The step of checking the percentage ratio between the quantity of the added dose of disinfecting substance with respect to the mixture is properly carried out in such a way as to maintain said ratio above a predetermined minimum value.

### BRIEF DESCRIPTION OF THE DRAWINGS

Further advantages, objects and characteristics, as well as further embodiments of the present invention are defined in the claims and will be illustrated in the following description, with reference to the enclosed drawings; in the drawings, corresponding or equivalent characteristics and/or components of the present invention are identified by the same reference numbers. In particular:
- Figure 1 shows an axonometric view of a piece of equipment according to a preferred embodiment of the invention;
- Figure 2 shows the operating diagram of the equipment shown in Figure 1;
- Figures from 3 to 6 show different operating stages of the equipment shown in Figure 1 with reference to the operating diagram of Figure 2;
- Figure 7 shows the operating diagram of a variant embodiment of the equipment of the invention.

### DETAILED DESCRIPTION OF THE PRESENT INVENTION

Although the present invention is described below with reference to its embodiments illustrated in the drawings, the present invention is not limited to the embodiments described below and illustrated in the drawings.

On the contrary, the embodiments described and illustrated in the drawings clarify some aspects of the present invention, the scope of which is defined in the claims.

The present invention can be especially but not exclusively used in the field of hospitals. In particular, the equipment for disinfecting organic substances that is the subject of the invention can advantageously be used during surgical operations. The words "organic substance" will thus preferably mean a liquid substance, for example blood or urine. Said liquid substance can be thicker or thinner, as well as containing larger or smaller suspended particles of a different type.

For this reason, the present invention is described here below with particular reference to its application during surgery. It should however be noted that the applications of the present invention are not limited to the case of surgery; on the contrary, the present invention can be advantageously applied in all those cases in which it is necessary to treat organic substances, preferably liquid, that are potentially infected. The equipment of the invention can be advantageously used, for example, in dental surgeries, general surgeries, private hospitals etc.

With reference to Figures from 1 to 6, here below is the description of a first embodiment of the equipment 1 for the disinfection of organic substances; in the figures, similar or equivalent characteristics and/or component parts are identified by the same reference numbers.

To simplify the present description, here below the equipment for disinfecting organic substances 1 that is the subject of the invention is simply defined as the equipment 1.

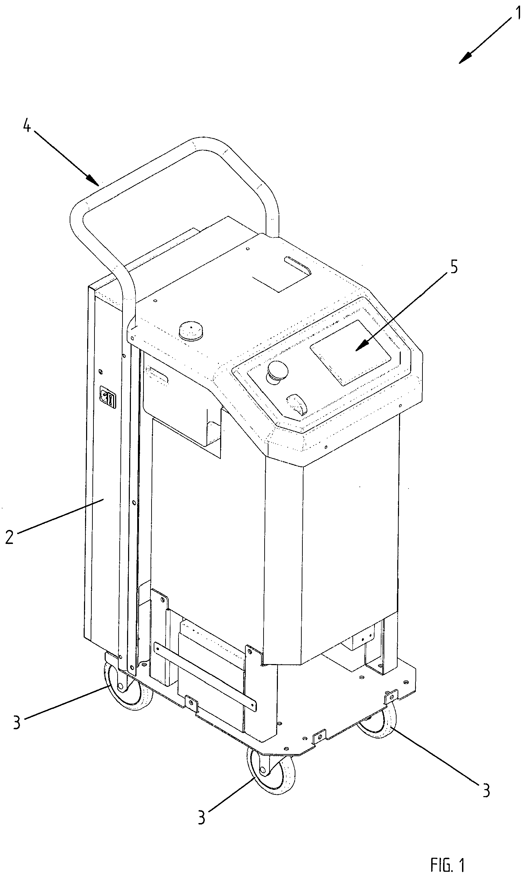

Figure 1 shows a preferred embodiment of the equipment 1 according to the present invention.

The equipment 1 is carried out according to a movable configuration and comprises a body 2, preferably made of thermoformed polystyrene, mounted on wheels 3. This makes the equipment 1 particularly resistant to impacts. The wheels 3 are preferably pivoting and antistatic wheels and are provided with brakes. In this way the equipment 1 can be comfortably transported and is protected against any electrostatic charges. An external manoeuvring handle 4 facilitates its movements.

A display 5, preferably of the touch-screen type, allows the various operating data to be input and displayed by the operator, as will be better explained below. The above mentioned characteristics, together with others described below, make the equipment 1 especially suited to be used in hospital environments, like wards or operating theatres.

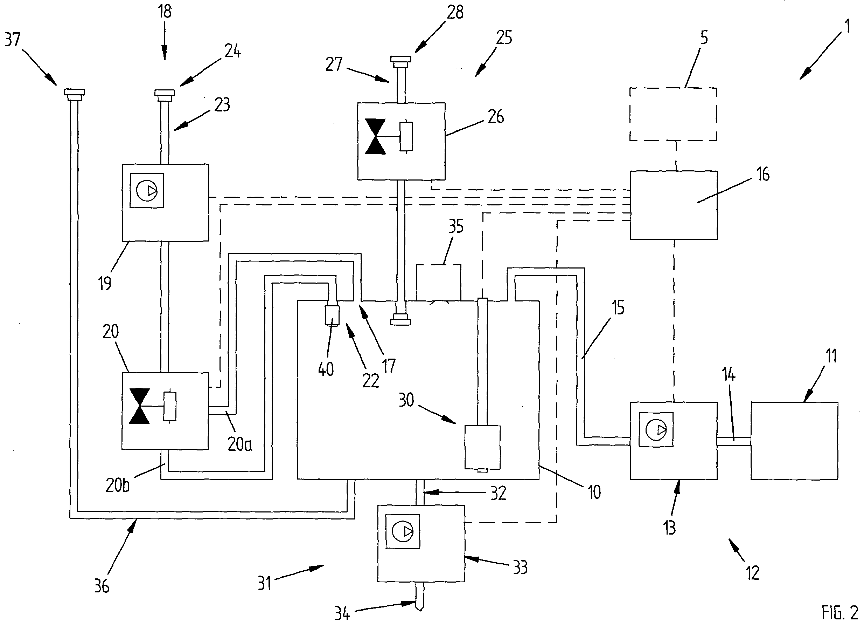

The equipment 1 of Figure 1 is described in detail with reference to the schematic view of Figure 2.

The equipment 1 comprises a main container 10 suited to receive the organic substance O to be treated.

The words "organic substance" O mean, in the continuation of this description, any type of substance, preferably liquid, which must necessarily be subjected to a treatment intended to neutralize any risk factors, for example bacteria carrying infectious diseases like hepatitis B, hepatitis C, HIV, HBV, HCV etc..

Said neutralization or disinfection treatment advantageously consists in the disinfection of the organic substance through the addition of a suitable disinfecting substance S. In order to perform its disinfecting action, the disinfecting substance S, as will be better highlighted below, must be present in a suitable percentage in the organic substance O to be treated and act for a predetermined time of action. Said time interval must exceed a minimum time required to ensure an effective disinfection.

The disinfecting substance S is advantageously received in a secondary container 11. Between the secondary container 11 and the main container 10 there are conveying means 12 suited to convey the disinfecting substance S into the main container 10 in a controlled manner.

Said conveying means 12 preferably comprise a peristaltic pump 13, a duct 14 for suction from the secondary container 11 and a conveying duct 15 for conveyance to the main container 10. A logic control unit 16 properly controls the peristaltic pump 13.

The controlled operation of the peristaltic pump 13 allows doses of the disinfecting substance S to be withdrawn from the secondary container 11 and introduced in the main container 10.

In variant embodiments of the invention, said conveying means may be of a different type but suitable for the intended purpose, for example they may be other types of pump.

The main container 10 is associated with a first inlet 17 for conveying the organic substance O to be treated. For this purpose the equipment 1 comprises an inlet circuit 18 for the introduction of the organic substance O, taken from the outside, into the main container 10.

The inlet circuit 18 preferably comprises an intake pump 19 connected to the first inlet 17 of the main container 10 through controlled opening/closing means 20. Said controlled opening/closing means 20 preferably comprise a two-way solenoid valve, the ways being indicated by 20a, 20b. The first outlet way 20a is connected to said first inlet 17 of the main container 10. The second outlet way 20b is connected to a second inlet 22 of the main container 10, whose function will be explained in greater detail below. Preferably, said second inlet 22 comprises a diffuser element 40, like for example a 360° rotary nozzle.

Both the intake pump 19 and the solenoid valve 20 are properly connected to the logic control unit 16.

The intake pump 19 is connected to an intake pipe 23 provided with an end connector 24.

During the operation of the equipment 1, an external intake duct will be properly connected to the end connector 24, as is better explained below.

The equipment 1 also comprises a circuit for conveying liquid substances 25. Said liquid substance conveying circuit 25 preferably comprises controlled opening/closing means 26, for example a solenoid valve, and a corresponding inlet duct 27 provided with an end connector 28.

The opening/closing means 26 are properly connected to the logic control unit 16.

Preferably, said conveying circuit 25 for liquid substances is connected to a water supply hydraulic circuit through the end connector 28.

The main container 10 is associated with means for detecting its filling level 30. In the embodiment illustrated herein the detection means 30 are arranged inside the main container 10 and are constituted by a magnetic reed float suited to slide vertically depending on the level of the substance present inside the container 10. The detection means 30 advantageously transmit the filling value of the main container 10 to the logic control unit 16.

According to variant embodiments of the invention, the means for detecting the contents of the main container may be of a different type, for example they may be optical detection means, flow sensors etc.

A discharge circuit 31 is associated with the main container 10. The discharge circuit 31 preferably comprises a suction duct 32 arranged on the bottom of the container 10, discharge means 33, preferably a discharge pump, and an outlet duct 34.

The discharge means 33 are advantageously connected to the logic control unit 16.

The outlet duct 34 may advantageously be connected to a discharge channel, if necessary, as for example the sewerage system.

The main container 10 is associated with an auxiliary container 35, communicating with the inside of the main container 10 and suited to receive a substance S' for cleaning and disinfecting the equipment 1. Advantageously, the cleaning and disinfecting substance S' comes in the form of single-dose capsules, as is better illustrated below.

The bottom of the main container 10 is associated with a recirculation duct 36 provided with an end connector 37.

As is better explained in the description of the operating steps of the equipment 1, the end connector 37 is suited to be connected to the end connector 24 of the intake pipe 23 of the inlet circuit 18 to obtain a closed recirculation circuit. Means 5 for displaying and inputting data, preferably in the form of a display, previously indicated with reference to Figure 1, are advantageously connected to the logic control unit 16.

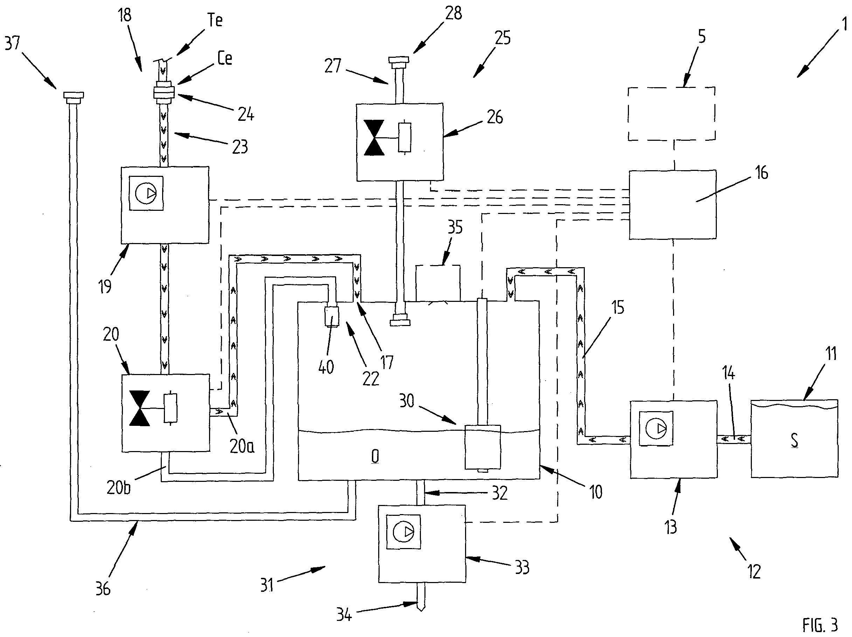

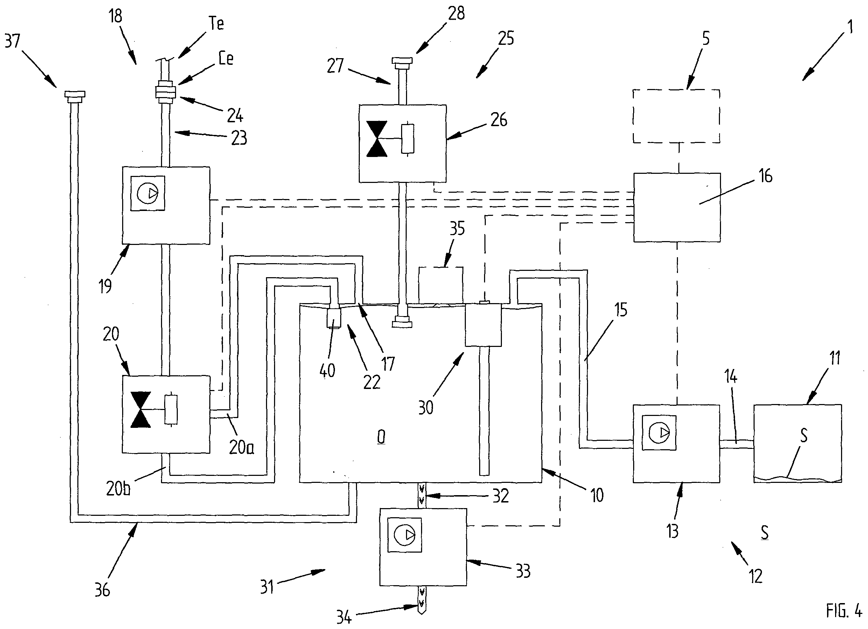

The operation of the equipment 1 of Figure 2 according to a preferred embodiment of the method of the invention is described below with reference to Figures 3 and 4.

The use of the equipment 1 includes, first of all, the connection of its inlet circuit 18 to the point of collection of the organic substance O to be treated. In particular, the end connector 24 of the intake pipe 23 is connected to a corresponding connector Ce of an external intake pipe Te, as shown in Figure 3. The external intake pipe Te can be associated, for example, with a bag for collecting liquids used for surgical operations.

A filter, not shown in the figure, is preferably interposed between the two connectors 24 and Ce. Said filter preferably comprises a filtering net and serves as a protection element for any organic material that may obstruct and/or damage the intake pump 19 of the inlet circuit 18.

Once the connection with the external intake pipe Te has been carried out, the equipment 1 is ready for operation.

During said operation, the organic substance O is withdrawn through the inlet circuit 18 and introduced in the main container 10.

For this purpose the intake pump 19 is operated and the two-way solenoid valve is properly controlled to convey the withdrawn organic substance O through the first outlet way 20a. The organic substance O reaches the main container 10 through the first inlet 17, as schematically shown by the arrows.

The control of the intake pump 19 and of the two-way solenoid valve 20 is advantageously performed by the logic control unit 16.

The operation of the intake pump 19 can be continuous, in the case of high quantities of organic substance O coming from the external intake pipe Te, or intermittent, or again controlled based on the flow coming from the external pipe Te. In this last case a suitable flow sensor can be associated with the inlet circuit 18.

Advantageously, the type of operation can be selected by the operator using, for example, the display 5 where a suitable page for setting the type of operation will be displayed or selectable for this purpose.

The organic substance O is thus introduced in the main container 10 and at the same time the detection means 30, in our case the magnetic reed float, detect its level. The value of the level is made available to the logic control unit 16. The logic control unit 16 can comfortably deduce from the detected level values the quantity of organic substance O present or added in the main container 10.

The logic control unit 16 thus acts on the conveying means 12 of the disinfecting substance S.

According to the invention, the logic control unit 16 activates the peristaltic pump 13 for the introduction of a dose of disinfecting substance S inside the main container 10 based on the quantity of organic substance O present in the main container 10. In particular, a dose D of disinfecting substance S is introduced in the main container 10 every time the organic substance O inside the main container 10 increases by a predetermined quantity Q. For example, a 5 cc dose D is added per each increase of the organic substance O by a quantity Q equal to 500 cc.

The increase of the organic substance O by said quantity Q can be easily calculated by the logic control unit 16 through the processing of the values detected with the magnetic reed float 30. The dose D of disinfecting substance S can be easily made available, for example, through a predetermined number of revolutions of the peristaltic pump 13.

According to the above, the quantity of disinfecting substance S inside the main container 10 will be maintained at a controlled percentage ratio R with respect to the organic substance O, for example 1% in the case indicated above. It is evident that in construction variants said percentage ratio R can be different. Said percentage ratio R may depend, for example, on the type of disinfecting substance S used. Preferably, said percentage ratio value R can be selected by the operator using, for example, the display 5 where a suitable page for setting said percentage ratio will be displayed or selectable for this purpose.

In a preferred embodiment of the invention, the capacities of the main container 10 and of the secondary container 11 are selected based on said percentage value. For example, a capacity of 62 1 can be selected for the main container 10 and a capacity of 3000 cc can be selected for the secondary container 11.

The capacity of the second container 11 is thus advantageously selected so that it exceeds 1% of the capacity of the first container 10, in such a way as to be able to accommodate the necessary disinfecting substance S.

The percentage ratio R between disinfecting substance S and organic substance O represents, as already explained, a very important parameter for the correct execution of the disinfecting process. In general, the equipment must ensure that said percentage ratio R is higher than or equal to a predefined minimum percentage ratio Rmin.

In addition to what has been said with reference to the parameter constituted by the percentage ratio R of disinfecting substance S in relation to the organic substance O, another fundamental control parameter for the effectiveness of the disinfecting action is the time of action ta of the disinfecting substance S in the organic substance O.

The time of action ta is monitored for this purpose by the control unit 16 that will provide for signalling, for example by means of a message on the display 5, when said time of action ta has elapsed.

Furthermore, special safety systems can be included to prevent access to the organic substance O before said time of action ta has elapsed. For example, an acoustic and/or visual signal can be provided.

Once the time of action ta has actually elapsed, inside the main container 10 there will be the mixture of disinfected organic substance O'.

The equipment 1 is thus ready for discharging said disinfected organic substance O'. This may preferably take place immediately or, alternatively, at a later moment. Preferably, said discharge operation takes place when the main container 10 is full. Said condition is detected by the control unit 16 through the magnetic reed float 30, as substantially shown in Figure 4.

The secondary container 11 will advantageously be empty, as already explained.

In a preferred embodiment of the invention a system for measuring the residual quantity of disinfecting substance S in the second container 11 can be provided, so as to ensure that all its contents have been actually discharged in the main container 10, wherein said system can comprise, for example, optical, magnetic, capacitive, inductive sensors etc..

In construction variants of the invention, the discharge operation can be carried out with any quantity of disinfected organic substance O' present in the main container 10, in this case taking care to guarantee in any case that the disinfecting substance S and the organic substance O are in the correct percentage ratio R and that the time of action ta has elapsed.

The discharge step takes place through the discharge circuit 31. The discharge pump 33 is operated and the disinfected organic substance O' is withdrawn through the suction duct 32 and conveyed into the outlet duct 34, as schematically shown by the arrows in Figure 4.

Advantageously, the outlet duct 34 can be directly connected to the sewerage system.

At this point the disinfection step can be considered completed.

The above clearly shows that the equipment 1 allows the set objects to be achieved.

Advantageously, in fact, the organic substance O is collected safely, in the case at end by the liquid collection bag used for surgical operations, and immediately introduced in the main container 10. Said main container 10 is made so that it is as tight as possible and advantageously insulated from the external environment. In this way any contact between the potentially infected organic substance O and the operators and the surrounding environment is avoided.

Furthermore, the organic substance O undergoes the disinfection treatment directly on site, for example during the surgical operation itself, with no need to store the organic substance O at the hospital while waiting for it to be collected in order to be successively treated as special waste.

Still advantageously, the possibility to treat the organic substance O directly on site allows it to be immediately discharged, for example by simply conveying the disinfected organic substance O' into the sewerage system.

This means reducing the hospital's disposal costs.

With reference to the description provided above and in particular with reference to Figure 4, it has been assumed that the main container 10 reaches the "full" condition naturally, that is, following successive introduction operations of organic substance O through the inlet circuit 18.

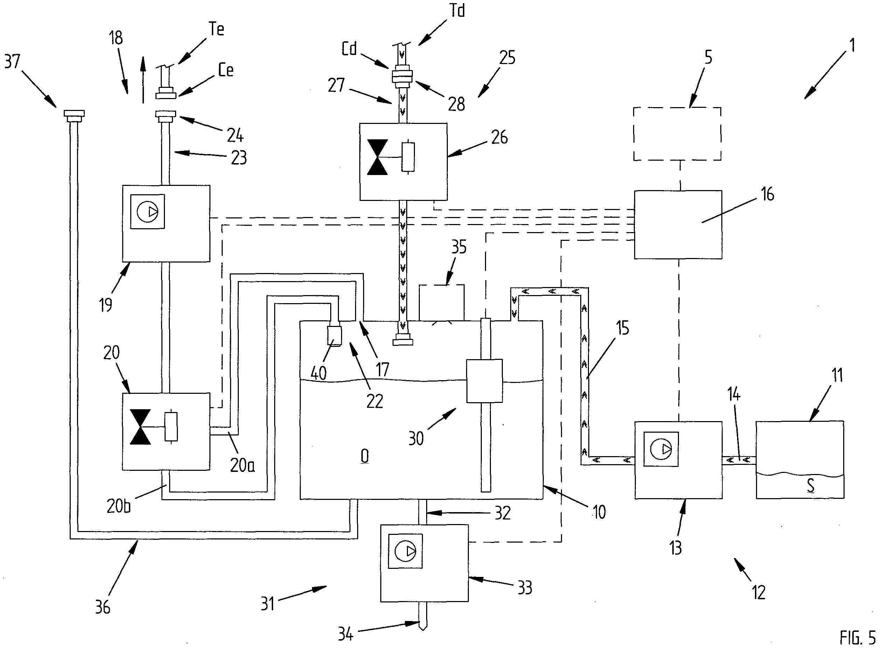

In practice, however, said condition may not occur. More frequently, in fact, the main container 10 is filled with a given quantity of organic substance O that reaches a level below the full load, as indicatively shown in Figure 5.

At the same time, inside the secondary container 11 there will be a residual quantity of disinfecting substance S.

According to a preferred operation mode of the equipment 1, instead of maintaining said condition of partial filling while waiting for successive introduction operations in order to fill the main container 10 completely, a finalization step is immediately carried out that makes it possible to proceed immediately and in any case to the disinfection and discharge of the organic substance O.

For this purpose, as shown in Figure 5, first of all the external intake pipe Te is disconnected from the inlet circuit 18.

Then the liquid substance conveying circuit 25 is activated. For this purpose, the end connector 28 of the inlet duct 27 is advantageously connected to the conveying pipe Td of a liquid L, advantageously water, through a respective connector Cd.

At this point the logic control unit 16 will provide for filling the main container 10 completely with the liquid L, operating the respective solenoid valve 26.

At the same time, all the residual disinfecting substance S present in the secondary container 11 is introduced in the main container 10.

In this way, to advantage, the percentage ratio R of disinfecting substance in relation to the quantity of material contained in the main container 10 is ensured, said material in this case being constituted by the mixture of organic substance O and filling liquid L.

Once the time of action ta has actually elapsed, it will be possible to proceed to the successive discharge step, according to the procedures described above.

The finalization step just described, including the use of a filling liquid L, advantageously allows the use of all the disinfecting substance S contained in the secondary container 11. This may be particularly advantageous if the disinfecting substance S has an expiry date.

Its delayed use may in fact involve a reduction in its disinfecting power, with evident risks for the effectiveness of the disinfection of the organic substance O.

According to a variant of the disinfecting method of the invention, the finalization step with the addition of filling liquid L may not be carried out immediately after the surgical operation but may be postponed, if necessary after successive uses of the equipment 1. In this case, however, the finalization step will be carried out automatically within the expiry date of the disinfecting substance S.

In this case, therefore, it will be possible to set the expiry date of the disinfecting substance S, always by means of the display 5 or, alternatively, the duration of the disinfecting substance S from the moment it is introduced in the secondary container 11.

Again, alternatively, said date may be detected automatically, for example by providing the equipment with an optical reader capable of detecting the expiry date or the duration on a respective bar code provided on the package of the disinfecting substance S.

At the end of the discharge step, as previously described, the equipment 1 carries out a disinfection step. This step has the purpose of cleaning and disinfecting the main container 10 and the inner circuits of the equipment 1 that have come into contact with the organic substance O.

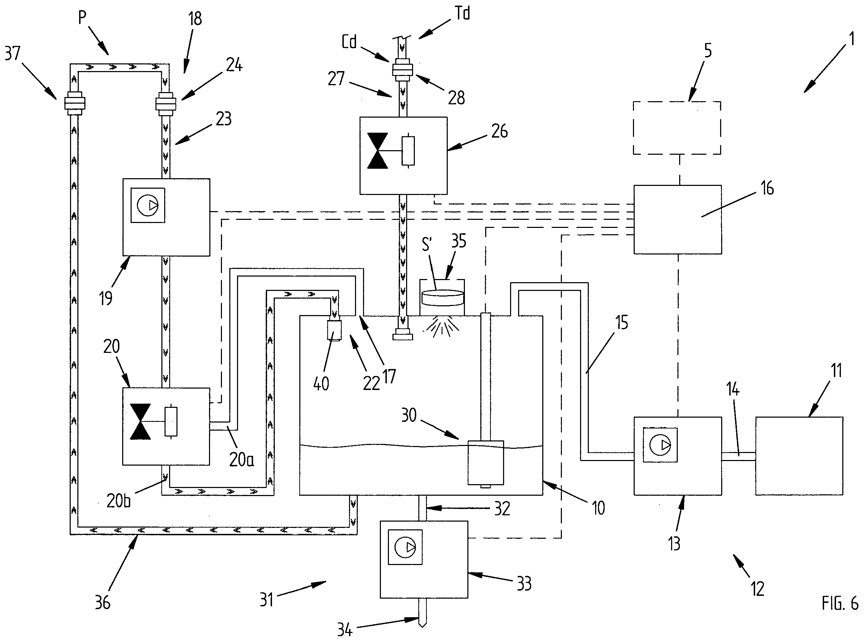

Said step is described with reference to Figure 6.

The disinfection step includes the use of a cleaning and disinfecting substance S'. The cleaning and disinfecting substance S' is positioned inside the special receiving seat 35. Advantageously, said cleaning and disinfecting substance S' comes in the form of single-dose capsules, for example 20 cc capsules.

The cleaning and disinfecting substance S' is thus introduced in the main container 10, which will result to be substantially empty following the previously performed discharge step.

A quantity of detergent liquid L, advantageously water, is thus introduced in the main container 10 through the liquid conveying circuit 25.

The quantity of detergent liquid L introduced will be such as to guarantee the required percentage ratio for the special cleaning and disinfecting substance S' used, for example 15 1 of detergent liquid L for said single dose of cleaning and disinfecting S' substance equal to 20cc.

At the same time, the recirculation duct 36 is connected to the intake pipe 23 of the inlet circuit 18. This can be done for example by an operator, making a bridge connection P between the two respective end connectors 37, 24 of the recirculation duct 36 and of the intake pipe 23, said end connectors 37, 24 being preferably made easily accessible on the body 2 of the equipment 1.

In this way a recirculation circuit is created, schematically indicated by the arrows in Figure 6 and starting from the bottom of the main container 10 to follow the recirculation duct 36, pass through the bridge P, the intake pipe 23, the intake pump 19 and then through the second way 20b of the solenoid valve 20 to reach the second inlet 22 of the main container 10.

The recirculation circuit is started by operating the intake pump 19.

The disinfecting mixture inside the main container 10, constituted by detergent liquid L and cleaning and disinfecting substance S', is therefore made flow inside the recirculation circuit and sprayed inside the main container 10 through the nozzle 22 at 360°. The nozzle 22 advantageously directs the disinfecting mixture on all the inner surfaces of the main container 10.

During said recirculation step also the pipes of the inlet circuit 18 are disinfected. Furthermore, by properly piloting the solenoid valve 20 towards the first way 20a it is possible to recirculate the disinfecting mixture even through the pipe that connects the first inlet 17 of the main container 10.

During the recirculation step, advantageously, a foam is created that makes it possible to improve the disinfecting properties of the disinfecting mixture.

The recirculation step extends for a preferred time interval that depends on the characteristics of the disinfecting mixture.

At the end of this operation, a discharge operation is performed by activating the discharge means 31 in order to eliminate the disinfecting mixture.

During the discharge step, or alternatively, it will be possible to introduce further quantities of detergent liquid L to rinse and remove the disinfecting mixture completely.

The equipment 1 will thus be ready for a successive use.

According to a preferred embodiment of the method, the detergent liquid L used is at ambient temperature.

In a further embodiment of the method, a heated detergent liquid L is used.

Advantageously, said heated detergent liquid L is supplied at said temperature from the outside. According to a variant embodiment, the equipment 1 may be provided with means for heating the liquid L coming from the outside, such as an electric resistance.

Advantageously, the equipment 1 carries out an automatic disinfection cycle in a simple and immediate manner.

The equipment 1 and the various parts that make it up and need power are preferably powered with low voltage batteries, for example 24V batteries, advantageously of the rechargeable type. This makes its use safe, in particular inside operating theatres.

In a preferred embodiment, instead, the discharge means are powered through the power mains, meaning at 220V. In this way it is possible to include the use of a discharge pump with sufficiently high flow rate, for example 20 litres/minute, so as to make the discharge step as rapid as possible.

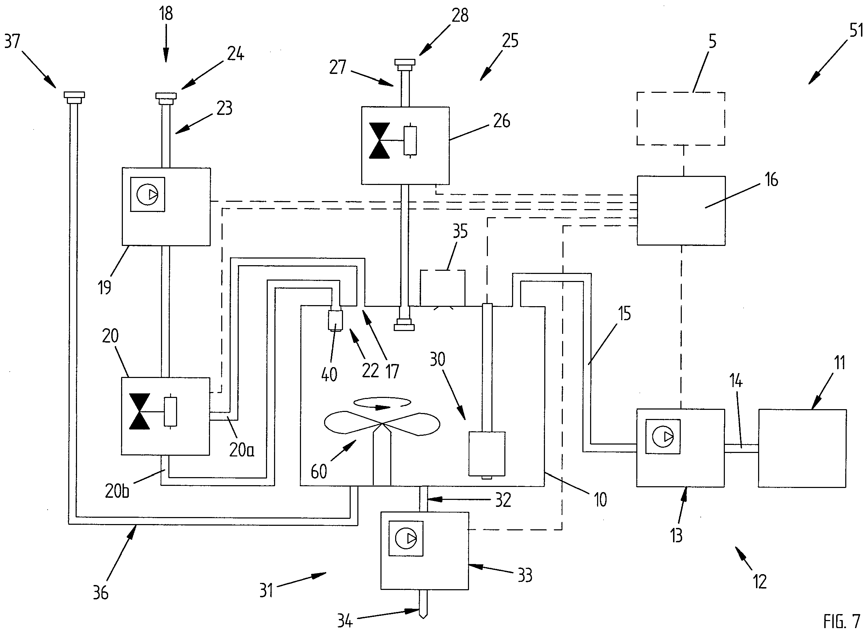

Figure 7 shows a construction variant of the equipment 51 of the invention.

This variant differs from the one described with reference to Figure 2 in that it comprises mixing means 60 inside the main container 10.

The mixing means 60, constituted for example by a propeller suitably set rotating, advantageously favour the mixing process between the organic substance O and the doses of disinfecting substance S introduced in the main container 10, thus improving the disinfecting action.

It has thus been shown that the invention described above achieves all the set objects.

In particular, the present invention makes it possible to solve the problems related to the collection and disinfection of organic substances.

While the present invention has been described with reference to the particular embodiments shown in the figures, it should be noted that the present invention is not limited to the specific embodiments illustrated and described herein; on the contrary, further variants of the embodiments described herein fall within the scope of the present invention, scope which is defined in the claims.

## Claims

1. Equipment (1; 51) for disinfecting a liquid organic substance (O) containing suspended particles, comprising:
- main containment means (10) comprising an inlet (17) for conveying said organic substance (O) to be treated;
- secondary containment means (11) suited to receive a disinfecting substance (S);
- means (12) for conveying said disinfecting substance (S) from said secondary containment means (11) to said main containment means (10), said conveying means comprising a pump (13);
- means (18) for the intake of said organic substance (O) associated with said conveying inlet (17);
**characterized in that** it comprises a logic control unit (16) configured to activate said pump (13) for the introduction of a dose (D) of said disinfecting substance (S) in said main containment means (10) every time said organic substance (O) inside said main containment means (10) increases by a predetermined quantity (Q).

2. Equipment (1; 51) according to claim 1, **characterized in that** it comprises means (30) for detecting the contents of said main containment means (10).

3. Equipment (1; 51) according to any of the preceding claims, **characterized in that** it comprises discharge means (31) associated with said main containment means (10) and for discharging the contents of said main containment means (10).

4. Equipment (1; 51) according to any of the preceding claims, **characterized in that** it comprises means (25) for conveying liquid substances associated with said main containment means (10).

5. Equipment (1; 51) according to any of the preceding claims, **characterized in that** it comprises recirculation means (36) associated with said main containment means (10) and for recirculating the substances contained in said main containment means (10).

6. Equipment (1; 51) according to claim 5, **characterized in that** said recirculation means (36) cooperate with said means (18) for the intake of said organic substance (O).

7. Equipment (1; 51) according to any of the preceding claims, **characterized in that** it comprises auxiliary containment means (35) suited to receive a cleaning and disinfecting substance (S') for disinfecting said equipment (1; 51).

8. Equipment (51) according to any of the preceding claims, **characterized in that** it comprises mixing means (60) associated with said main containment means (10).

9. Method for disinfecting a liquid organic substance (O) containing suspended particles, **characterized in that** it is carried out using an equipment according to any of the preceding claims, said method comprising the following steps:
- introducing a quantity of said organic substance (O) to be disinfected into said main containment means (10) by means of said means (18) for the intake of said organic substance (O) associated with said conveying inlet (17);
- conveying a dose (D) of said disinfecting substance (S) from said secondary containment means (11) to said main containment means (10) and adding by means of said pump (13) of said conveying means (12), said dose (D) of said disinfecting substance (S) to said organic substance (O) every time said organic substance (O) increases in said main containment means (10) by a predetermined quantity (Q);
- maintaining said organic substance (O) to be disinfected and said dose of disinfecting substance (S) in contact in order to obtain a disinfected substance (O');
- discharging said disinfected substance (O') from said main containment means (10).

10. Method according to claim 9), **characterized in that** said step of maintaining said organic substance (O) and said dose of disinfecting substance (S) in contact takes place at least for a predetermined minimum time of action (ta).

11. Method according to claim 9) or 10), **characterized in that** said step of conveying a dose of a disinfecting substance (S) comprises the step of checking the percentage ratio (R) between the quantity of said conveyed dose of said disinfecting substance (S) with respect to said organic substance (O) to be disinfected, in such a way as to maintain said ratio above a predetermined minimum value (Rmin).

12. Method according to any of the claims from 9) to 11), **characterized in that** it comprises a step during which a quantity of a filling substance (L) is introduced in said main containment means (10) in order to obtain a mixture.

13. Method according to claim 12), **characterized in that** it comprises a further step of adding a dose of a disinfecting substance (S) in said main containment means (10) and **in that** it comprises a step of checking the percentage ratio (R) between the quantity of said conveyed dose of said disinfecting substance (S) with respect to said mixture.

14. Method according to claim 13), **characterized in that** said step of checking the percentage ratio (R) between the quantity of said conveyed dose of said disinfecting substance (S) with respect to said mixture (S) is carried out in such a way as to maintain said ratio (R) above a predetermined minimum value (Rmin).

## Patentansprüche

1. Ausrüstung (1; 51) zur Desinfektion einer flüssigen, organischen Substanz (O), die suspendierte Partikel enthält, Folgendes umfassend:
- Hauptbehältnismittel (10), einen Einlass (17) zur Leitung der besagten, zu behandelnden organischen Substanz (O) umfassend;
- sekundäre Behältnismittel (11, dazu geeignet, eine Desinfektionssubstanz (S) aufzunehmen;
- Mittel (12) zur Leitung der besagten Desinfektionssubstanz (S) aus dem besagten, sekundären Behältnismittel (11) in das besagte Hauptbehältnismittel (10), wobei die besagten Leitmittel eine Pumpe (13) umfassen;
- Mit dem besagten Leiteinlass (17) verbundene Mittel (18) zur Ansaugung der besagten organischen Substanz (O);
**dadurch gekennzeichnet, dass** sie eine Steuerlogikeinheit (16) umfasst, die so konfiguriert ist, dass sie die besagte Pumpe (13) aktiviert, um jedes Mal dann eine Dosis (D) der besagten Desinfektionssubstanz (S) in das besagte Hauptbehältnismittel (10) einzuführen, wenn die besagte organische Substanz (O) in dem besagten Hauptbehäitnismittel (10) um eine vorbestimmte Menge (Q) zunimmt.

2. Ausrüstung (1; 51) gemäß Patentanspruch 1, **dadurch gekennzeichnet, dass** sie Mittel (30) zur Erkennung des Inhalts des besagten Hauptbehältnismittels (10) umfasst.

3. Ausrüstung (1; 51) gemäß eines jeden der vorstehenden Patentansprüche, **dadurch gekennzeichnet, dass** sie mit dem besagten Hauptbehältnis verbundene Entlademittel (31) zur Entladung des Inhalts des besagten Hauptbehältnismittels (10) umfasst.

4. Ausrüstung (1; 51) gemäß eines jeden der vorstehenden Patentansprüche, **dadurch gekennzeichnet, dass** sie mit dem besagten Hauptbehältnismittel (10) verbundene Mittel (25) zur Leitung flüssiger Substanzen umfasst.

5. Ausrüstung (1; 51) gemäß eines jeden der vorstehenden Patentansprüche, **dadurch gekennzeichnet, dass** sie mit dem besagten Hauptbehältnismittel (10) verbundene Rezirkulationsmittel (36) für die Rezirkulation der in dem besagten Hauptbehältnismittel (10) enthaltenen Substanzen umfasst.

6. Ausrüstung (1; 51) gemäß Patentanspruch 5, **dadurch gekennzeichnet, dass** die besagten Rezirkulationsmittel (36) mit den besagten Mitteln (18) für die Ansaugung der besagten organischen Substanz (O) zusammenwirken.

7. Ausrüstung (1; 51) gemäß eines jeden der vorstehenden Patentansprüche, **dadurch gekennzeichnet, dass** sie zusätzliche Behältnismittel (35) umfassen, die geeignet sind, eine Reinigungs- und Desinfektionssubstanz (S') für die Desinfektion der besagten Ausrüstung (1, 51) aufzunehmen.

8. Ausrüstung (51; 51) gemäß eines jeden der vorstehenden Patentansprüche, **dadurch gekennzeichnet, dass** sie Mischmittel (60) umfasst, die mit dem besagten Hauptbehältnismittel (10) verbunden sind.

9. Verfahren zur Desinfektion einer flüssigen, organischen Substanz (O), die suspendierte Partikel enthält, **dadurch gekennzeichnet, dass** es unter Einsatz einer Ausrüstung gemäß eines jeglichen der vorstehenden Patentansprüche ausgeführt ist, wobei das besagte Verfahren die folgenden Schritte umfasst:
- Einleitung einer Menge der besagten, organischen und zu desinfizierenden Substanz (O) in das besagte Behältnismittel (10) durch die mit dem besagten Leiteinlass (17) verbundenen Mittel (18) für die Ansaugung der besagten organischen Substanz (O);
- Leitung einer Dosis (D) der besagten Desinfektionssubstanz (S) aus dem besagten sekundären Behältnismittel (11) in das besagte Hauptbehältnismittel (10) und Zugabe, mittels der besagten Pumpe (13) der besagten Leitmittel (12), der besagten Dosis (D) der besagten Desinfektionssubstanz (S) zu der besagten organischen Substanz (O) jedes Mal dann, wenn die besagte organische Substanz (O) in dem besagten Hauptbehältnismittel (10) um eine vorbestimmte Menge (Q) zunimmt;
- Erhalt des Kontakts zwischen der besagten organischen, zu desinfizierenden Substanz (O) und der besagten Dosis Desinfektionssubstanz (S), um eine desinfizierte Substanz (O') zu erhalten;
- Entladen der besagten, desinfizierten Substanz (O') aus dem besagten Hauptbehältnismittel (10).

10. Verfahren gemäß Patentanspruch 9), **dadurch gekennzeichnet, dass** der besagte Schritt des Erhalts des Kontakts zwischen der besagten organischen Substanz (O) und der besagten Dosis Desinfektionssubstanz (S) über wenigstens eine vorbestimmte Mindest-Wirkungszeit (ta) stattfindet.

11. Verfahren gemäß Patentanspruch 9) oder 10), **dadurch gekennzeichnet, dass** der besagte Schritt der Leitung einer Dosis einer Desinfektionssubstanz (S) den Schritt der Feststellung des prozentualen Verhältnisses (R) zwischen der Menge der besagten, geleiteten Desinfektionssubstanz (S) und der besagten organischen, zu desinfizierenden Substanz (O) umfasst, so dass das besagte Verhältnis oberhalb eines vorbestimmten Mindestwerts (Rmin) gehalten wird.

12. Verfahren gemäß eines jeden der Patentansprüche von 9 bis 11, **dadurch gekennzeichnet, dass** es einen Schritt umfasst, während dessen eine Menge einer Füllsubstanz (L) in das besagte Hauptbehältnismittel (10) eingeführt wird, um eine Mischung zu erhalten.

13. Verfahren gemäß Patentanspruch 12), **dadurch gekennzeichnet, dass** es einen weiteren Schritt der Zugabe einer Dosis Desinfektionssubstanz (S) in das besagte Hauptbehältnismittel (10) umfasst und dadurch, dass es einen Schritt der Feststellung des prozentualen Verhältnisses (R) zwischen der Menge der besagten, geleiteten Dosis Desinfektionssubstanz (S) und der besagten Mischung umfasst.

14. Verfahren gemäß Patentanspruch 13), **dadurch gekennzeichnet, dass** der besagte Schritt der Feststellung des prozentualen Verhältnisses (R) zwischen der Menge der besagten, geleiteten Dosis der besagten Desinfektionssubstanz (S) und der besagten Mischung (S) so ausgeführt wird, dass das besagte Verhältnis (R) oberhalb eines vorbestimmten Mindestwerts (Rmin) gehalten wird.

## Revendications

1. Équipement (1 ; 51) pour la désinfection d'une substance organique (O) contenant des particules suspendues, comprenant :
- des moyens de confinement principaux (10) comprenant une entrée (17) d'adduction de ladite substance organique (O) à traiter ;
- des moyens de confinement secondaires (11) aptes à recevoir une substance désinfectante (S) ;
- des moyens d'adduction (12) de ladite substance désinfectante (S) desdits moyens de confinement secondaires (11) auxdits moyens de confinement principaux (10), lesdits moyens de confinement comprenant une pompe (13) ;.
- des moyens (18) pour l'aspiration de ladite substance organique (O) associés avec ladite entrée d'adduction (17) ;
**caractérisé en ce qu**'il comprend une unité de contrôle logique (16) configurée de manière à actionner ladite pompe (13) pour l'introduction d'une dose (D) de ladite substance désinfectante (S) dans lesdits moyens de confinement principaux (10) chaque fois que ladite substance organique (O) à l'intérieur desdits moyens de confinement principaux (10) augmente d'une quantité prédéterminée (Q).

2. Équipement (1 ; 51) selon la revendication 1, **caractérisé en ce qu**'il comprend des moyens de détection (30) du contenu à l'intérieur desdits moyens de confinement principaux (10).

3. Équipement (1 ; 51) selon l'une quelconque des revendications précédentes, **caractérisé en ce qu**'il comprend des moyens de déchargement (31) associés auxdits moyens de confinement principaux (10) et pour décharger le contenu desdits moyens de confinement principaux (10).

4. Équipement (1 ; 51) selon l'une quelconque des revendications précédentes, **caractérisé en ce qu**'il comprend des moyens d'adduction de substances liquides (25) associés auxdits moyens de confinement principaux (10).

5. Équipement (1 ; 51) selon l'une quelconque des revendications précédentes, **caractérisé en ce qu**'il comprend des moyens de recirculation (36) associés auxdits moyens de confinement principaux (10) et pour la recirculation des substances contenues dans lesdits moyens de confinement (10).

6. Équipement (1 ; 51) selon la revendication 5, **caractérisé en ce que** lesdits moyens de recirculation (36) coopèrent avec lesdits moyens d'aspiration (18) de ladite substance organique (O).

7. Équipement (1 ; 51) selon l'une quelconque des revendications précédentes, **caractérisé en ce qu**'il comprend des moyens de confinement auxiliaires (35) aptes à recevoir une substance de nettoyage et de désinfection (S') pour la désinfection dudit équipement (1 ; 51).

8. Équipement (51) selon l'une quelconque des revendications précédentes, **caractérisé en ce qu**'il comprend des moyens de mélange (60) associés auxdits moyens de confinement principaux (10).

9. Méthode pour la désinfection d'une substance organique liquide (O) contenant des particules suspendues, **caractérisée en ce qu'**elle est exécutée en utilisant un équipement selon l'une quelconque des revendications précédentes, ladite méthode comprenant les phases suivantes :
- l'introduction d'une quantité de ladite substance organique (O) à désinfecter dans lesdits moyens de confinement principaux (10) grâce auxdits moyens (18) pour l'aspiration de ladite substance organique (O) associés avec ladite entrée d'adduction (17) ;
- le transport d'une dose (D) de ladite substance désinfectante (S) desdits moyens de confinement secondaires (11) auxdits moyens de confinement principaux (10) et ajout au moyen de ladite pompe (13) desdits moyens de transport (12) de ladite dose (D) de ladite substance désinfectante (S) à ladite substance organique (O) chaque fois que ladite substance organique (O) augmente dans lesdits moyens de confinement principaux (10) d'une quantité prédéterminée (Q) ;
- le maintien en contact de ladite substance organique (O) à désinfecter et de ladite dose de substance désinfectante (S) pour obtenir une substance désinfectée (O') ;
- la vidange de ladite substance désinfectante (O') desdits moyens de confinement principaux (10).

10. Méthode selon la revendication 9), **caractérisé en ce que** ladite phase de maintien en contact de ladite substance organique (O) et de ladite dose de substance désinfectante (S) se vérifie au moins pendant un temps d'action minimal prédéterminé (tₐ).

11. Méthode selon la revendication 9) ou 10), **caractérisé en ce que** ladite phase de transport d'une dose d'une substance désinfectante (S) comprend la phase de contrôle du rapport de pourcentage (R) entre la qualité de ladite dose transportée de ladite substance désinfectante (S) par rapport à ladite substance organique (O) à désinfecter, de façon à maintenir ce rapport au-dessus d'un rapport minimal préfixé (Rmin).

12. Méthode selon l'une quelconque des revendications de 9) à 11), **caractérisé en ce qu**'elle comprend une phase d'introduction d'une quantité d'une substance de remplissage (L) dans lesdits moyens de confinement principaux (10) pour obtenir un mélange.

13. Méthode selon la revendication 12), **caractérisée en ce qu**'elle comprend une phase ultérieure d'ajout d'une dose d'une substance désinfectante (S) dans lesdits moyens de confinement principaux (10) et qu'elle comprend une phase de contrôle du rapport de pourcentage (R) entre la quantité de ladite dose transportée de ladite substance désinfectante (S) par rapport audit mélange.

14. Méthode selon la revendication 13), **caractérisée en ce que** ladite phase de contrôle du rapport de pourcentage (R) entre la quantité de ladite dose transportée de ladite substance désinfectante (S) par rapport audit mélange (S) est réalisée de façon à maintenir ce rapport (R) au-dessus d'un rapport minimal préfixé (Rmin).
